# NEUE EUROPÄISCHE PATENTSCHRIFT

(11) **EP 0 351 542 B2**
(45) Veröffentlichungstag und Bekanntmachung des Hinweises auf die Entscheidung über den Einspruch: **13.12.1995**
(45) Hinweis auf die Patenterteilung: 12.08.1992
(21) Anmeldenummer: 89110547.0
(22) Anmeldetag: 10.06.1989
(51) Int. Cl.: A61K 9/68, A61K 9/20, A61K 47/00

(54) **Xylit- und zuckerfreie Kautablette**
Xylite and sugar-free chewing tablet
Comprimé à mâcher sans sucre ou xylite

(30) Priorität: 18.07.1988 DE 3824318
(43) Veröffentlichungstag der Anmeldung: 24.01.1990
(73) Patentinhaber: MERCK PATENT GmbH, D-64271 Darmstadt (DE)
(72) Erfinder: Starz, Carola, D-6200 Wiesbaden (DE); Stieber, Herbert, D-6103 Griesheim (DE); Färber, Dagmar, D-6147 Lautertal 1 (DE)

(56) Entgegenhaltungen:
- DE-A- 2 658 282
- FR-A- 2 101 009
- LU-A- 60 738
- US-A- 3 655 866
- US-A- 4 233 288
- US-A- 4 248 895
- US-A- 4 892 889
- PATENT ABSTRACTS OF JAPAN vol. 8, no. 34 (C-210)(1471) 15 Februar 1984
- "Pharmaceutical Dosage Forms", vol. 1, Herbert A. Liebermann, Leon Lachmann, New York und Basel, 1980
- Datenblatt Firma Roquette, Aug. 1986
- "Lehrbuch der pharmazeutischen Technologie", R. Voigt, 1984
- "Technische Information", Merck Darmstadt
- "Pharmazeutische technologie". G. Thieme Verlag, 1978
- "Drug development and industrial pharmacy", 12 (11-13), 1986

## Beschreibung

Die Erfindung betrifft xylit- und zuckerfreie Kautabletten auf Sorbitbasis, die ein Lockerungsmittel enthalten. Die als Lockerungsmittel verwendeten Stoffe weisen vorzugsweise wertvolle ernährungsphysiologische Eigenschaften auf.

Zuckerhaltige Tabletten oder Bonbons zum Lutschen oder Kauen auf Basis von Saccharose oder Glucose sind schon seit langem bekannt. Sie fördern jedoch bei häufigem Genuß Karies und sind für bestimmte Personengruppen, z.B. Diabetiker, völlig ungeeignet. Um diese Nachteile zu eliminieren, bietet die Süßwaren-Branche seit einigen Jahren entsprechende Ware mit Zuckeraustauschstoffen wie Sorbit oder Xylit an.

Es ist aber bekannt, daß bei der Herstellung vorwiegend sorbithaltiger Tabletten ein hoher Preßdruck aufgewendet werden muß. Dies führt zur Bildung sehr harter, spröder, nicht mehr kaubarer Tabletten. Andererseits lassen sich vorwiegend xylithaltige Tabletten nicht in befriedigender Qualität herstellen, zumal sie noch andere unerwünschte Eigenschaften aufweisen.

Aus der DE-PS 26 58 282 sind kaubare Tabletten bekannt, die sowohl Xylit als auch ein Polyol, vorzugsweise Sorbit, enthalten. Die bevorzugten Tablettenmassen bestehen hier aus 30-60 % Xylit und 30-60 % Sorbit. Der Gesamtanteil an Polyol (Sorbit + Xylit) beträgt mindestens 85 %, meist aber über 90 %. Derartige Tablettenmassen lassen sich zwar gut tablettieren, und die so hergestellten Tabletten sind kaubar, sie weisen jedoch einige beträchtliche Nachteile auf. So wird beispielsweise der durch den relativ hohen Xylitanteil bedingte starke Kühleffekt desöfteren als unangenehm empfunden. Zudem stört er bei vielen Aromatisierungen solcher Tablettenmassen, so daß eine größere Anzahl von gewünschten beigemengten Geschmacks- und Aromastoffen nicht oder nur ungenügend ihre volle Wirkung entfalten kann. Der sehr hohe Gesamtanteil an Zuckeralkohol bewirkt außerdem einen unerwünschten laxierenden Effekt, der insbesondere bei häufigerem Genuß nicht vernachlässigt werden kann. Weiterhin verhindert der für derartige Tabletten notwendige hohe Polyolanteil die Beimengung größerer Mengen weiterer wertvoller Stoffe. Letztlich sollte nicht unerwähnt bleiben, daß Xylit etwa viermal so teuer wie Sorbit ist, und daß somit auch aus ökonomischen Gründen eine Reduzierung von Xylit wünschenswert ist.

Der Erfindung liegt somit die Aufgabe zugrunde, eine neue, einfach zu tablettierende, haltbare, zuckerfreie, gut kaubare Tablette zu schaffen, die die durch Xylit bedingten negativen Eigenschaften nicht mehr aufweist.

Überraschend wurde nun gefunden, daß eine sehr gut verpreßbare, zuckerfreie, gut schmeckende und zahnschonende Kautablette auf Sorbitbasis erhalten werden kann, wenn man Xylit vollständig ersetzt durch Lockerungsmittel, die vorzugsweise ernährungsphysiologisch wertvoll sind, wie z.B. Eiweißstoffe, Mineralsalze, natürliche Ballaststoffe oder vergleichbare Materialien.

Gegenstand der Erfindung ist somit eine xylit- und zuckerfreie Kautablette auf Sorbitbasis, die ein Lockerungsmittel enthält.

Gegenstand der Erfindung sind insbesondere solche xylitund zuckerfreien Kautabletten auf Sorbitbasis, die als Lockerungsmittel Eiweiße oder Eiweißhydrolysate und/oder Mineralsalze enthalten.

Ferner ist Gegenstand der Erfindung ein Verfahren zur Herstellung der erfindungsgemäßen Kautabletten, das dadurch gekennzeichnet ist, daß man Sorbit mit dem Lockerungsmittel und gegebenenfalls mit lebensmittelrechtlich zugelassenen Stoffen vermischt und direkt zur Kautablette verpreßt.

Die erfindungsgemäßen Kautabletten enthalten als Lockerungsmittel vorzugsweise natürliche Substanzen wie Eiweiß, Mineralsalze, natürliche Ballaststoffe oder auch Kakaopulver. Vorzugsweise werden als Lockerungsmittel Eiweißstoffe oder Mineralsalze eingesetzt.

Als Eiweißstoffe eignen sich erfindungsgemäß alle Proteine und Proteinhydrolysate pflanzlichen oder tierischen Ursprungs. Als Beispiele sind Gelatine, Casein, Caseinate, Molke- oder Sojaproteine und (Mager-) Milchpulver zu nennen. Vorzugsweise wird Casein bwz. Caseinate, Magermilchpulver oder Gelatinehydrolysat verwendet. Erfindungsgemäß können auch Mischungen von verschiedenen Eiweißstoffen bzw. -hydrolysaten eingesetzt werden, um somit optimale sensorische Eigenschaften zu erzielen.

Der Gehalt an Eiweiß bzw. Eiweißhydrolysat variiert erfindungsgemäß zwischen 2 und 40 %, vorzugsweise zwischen 3 und 15 %, insbesondere zwischen 5 und 10 %. Das Verhältnis zwischen Sorbit als Basiskomponente der erfindungsgemäßen Kautablette und Eiweißstoff variiert zwischen 20:1 und 5:1, vorzugsweise zwischen 15:1 und 8:1.

Erfindungsgemäß können als Lockerungsmittel auch Mineralsalze eingesetzt werden. Hierbei kommen bevorzugt solche zum Einsatz, die einen positiven Einfluß auf den menschlichen Ionenhaushalt ausüben, so z.B. für den menschlichen Verzehr geeignete anorganische oder organische Natrium-, Kalium-, Calcium-, Magnesium- oder Eisen-Salze, z.B. Carbonate, Bicarbonate, Phosphate, Hydrogenphosphate, Sulfate, Chloride, Fluoride, Citrate oder Lactate. Bevorzugt sind hier die Carbonate von Calcium und Magnesium, die Bicarbonate und Citrate von Natrium und Kalium aber auch Magnesiumphosphat. Der gewichtsmäßige Anteil der Mineralsalze variiert zwischen 5 und 45 %, vorzugsweise zwischen 20 und 40 %. Das Verhältnis Sorbit zu Mineralsalz liegt bevorzugt zwischen 3:1 und ca. 1,5:1. Selbstverständlich sind auch Mischungen verschiedener Mineralsalzanteile geeignet.

Neben Eiweißstoffen und Mineralsalzen können auch natürliche Ballaststoffe wie feingemahlene Soja-, Mais- oder Weizen-Kleie oder auch Getreideschrot erfindungsgemäß als Lockerungsmittel zum Einsatz kommen. Bevorzugt wird aufgrund geschmacklicher und farblicher Kriterien Sojakleie. Der Gehalt an Ballaststoff variiert erfindungsgemäß zwischen 2 und 40 %, vorzugsweise zwischen 3 und 15 %, insbesondere zwischen 5 und 10 %. Das Verhältnis zwischen Sorbit als Basiskomponente der erfindungsgemäßen Kautablette und Ballaststoff variiert zwischen 20:1 und 5:1, vorzugsweise zwischen 15:1 und 8:1.

Als weiteres Lockerungsmittel für die erfindungsgemäße Kautablette eignet sich auch Kakaopulver, welches bevorzugt einen gewichtsmäßigen Anteil von 5 bis 30 % aufweist.

Erfindungsgemäß können auch Mischungen verschiedenartiger Lockerungsmittel, also Eiweiß und Mineralsalz, Eiweiß und Ballaststoff, Mineralsalz und Kakaopulver, Eiweiß und Ballaststoff und Mineralsalz oder andere mögliche Kombinationen verwendet werden, wobei der Gesamtanteil an Lockerungsmittel auch hier zwischen 2 und 45 % liegt.

Neben Sorbit als Basiskomponenten und Lockerungsmittel kann die Kautablette der Erfindung gegebenenfalls übliche lebensmittelrechtlich zugelassene Stoffe, wie natürliche, naturidentische oder künstliche Aroma- oder Geschmacksstoffe, Vitamine, Spurenelemente, Farbstoffe, Gleit- bzw. Trennmittel, künstliche Süßstoffe, Stabilisatoren, Antioxidantien enthalten. Der Anteil dieser Stoffe an der ganzen Kautablette liegt vorzugsweise zwischen 0,1 und 10 %.

Zur Herstellung der erfindungsgemäßen Kautablette werden die einzelnen Bestandteile (Sorbit, Lockerungsmittel und ggf. weitere Stoffe) in vorzugsweise trockener Form miteinander gemischt und in herkömmlichen Tablettiermaschinen gepreßt. Bei Beimischung von kleinen Mengen von Zusätzen erfolgt zur Erzielung eines besseren Mischungsgrades vorzugsweise eine Vormischung, bestehend aus den Zusatzkomponenten und einem Teil des Sorbits. Diese Vormischung wird dann mit dem Lockerungsmittel vermischt. Flüssige Komponenten werden ggf. auf die trockenen Bestandteile aufgesprüht.

### Beispiel 1

In einem Nautamischer werden 430 kg Sorbit, 25 kg Calcium-Caseinat, 25 kg Ascorbinsäure, 10 kg Geschmacksstoffe, 5 kg Aromastoffe und 5 kg Gleit- und Trennmittel (Magnesiumstearat, gehärtetes Fett) trocken miteinander vermischt und in einer handelsüblichen Tablettiermaschine verpreßt. Man erhält wohlschmeckende, sehr gut kaubare Tabletten mit sehr geringem Abrieb.

### Beispiel 2

Gemäß Beispiel 1 wird eine Tablettenmischung hergestellt und zu kaubaren Tabletten verpreßt, bestehend aus 414 kg Sorbit, 35 kg Magermilchpulver, 33 kg Vitaminmischung, 11 kg Aroma- und Geschmacksstoffe, 2 kg Farbstoff und 5 kg Trenn- und Gleitmittel.

### Beispiel 3

Analog Beispiel 1 wird eine Tablettenmischung hergestellt und tablettiert, bestehend aus 362 kg Sorbit, 40 kg Calciumcarbonat, 30 kg Magnesiumphosphat, 58 kg Kaliumcitrat, 5 kg Aroma- und Geschmacksstoff und 5 kg Trennund Gleitmittel.

### Beispiel 4

Analog Beispiel 1 wird eine Tablettenmischung hergestellt und zu Kautabletten verpreßt, bestehend aus 400 kg Sorbit, 45 kg Sojakleie, 30 kg Ascorbinsäure, 15 kg Geschmacksstoff, 5 kg Aromen und 5 kg Trenn- und Gleitmittel.

### Beispiel 5

Analog Beispiel 1 wird eine Mischung hergestellt und zu kaubaren Tabletten verpreßt, bestehend aus 400 kg Sorbit, 35 kg Gelatinehydrolysat, 10 kg Sojakleie, 33 kg Vitaminmischung, 11,5 kg Aroma- und Geschmacksstoff, 2,5 kg Farbstoff und 7,5 kg Trenn- und Gleitmittel.

### Beispiel 6

Gemäß Beispiel 1 wird folgende Mischung hergestellt und tablettiert: 325 kg Sorbit, 140 kg Calciumcarbonat, 25 kg Kakaopulver, 2,5 kg Geschmacksstoff und 7,5 kg Trennund Gleitmittel.

## Patentansprüche

1. Xylit- und zuckerfreie Vitamine enthaltende Kautablette auf Sorbitbasis für den Lebensmittelbereich, dadurch gekennzeichnet, daß sie ein Lockerungsmittel in Form von Eiweißen oder Eiweißhydrolysaten mit einem Gehalt von 2 bis 40% oder Mineralsalzen aus der Gruppe der Carbonate, Bicarbonate, Phosphate, Hydrogenphosphate, Sulfate, Chloride und Fluoride von Natrium, Kalium, Calcium, Magnesium oder Eisen mit einem Gehalt von 5 bis 40% enthält.

2. Kautablette nach Anspruch 1 mit einem Gehalt an Eiweißen bzw. Eiweißhydrolysaten von 3 bis 15% oder einem Gehalt an Salzen von 5 bis 20%.

3. Kautablette nach einem der Ansprüche 1 bis 2, dadurch gekennzeichnet, daß sie Vitamine mit einem Gehalt von 0.1 bis 10% enthält.

4. Verfahren zur Herstellung einer Kautablette gemäß einem der Ansprüche 1 bis 3, dadurch gekennzeichnet, daß man Sorbit mit dem Lockerungsmittel, den Vitaminen und gegebenenfalls mit weiteren lebensmittelrechtlich zugelassenen Stoffen vermischt und direkt zur Kautablette verpreßt.

5. Verwendung von Eiweißen, Eiweißhydrolysaten oder Mineralsalzen, als Lockerungsmittel in xylit- und zuckerfreien Kautabletten mit einem Gehalt an Eiweißstoffen von 2 bis 40% oder einem Gehalt an Mineralsalzen gemäß Anspruch 1 von 5 bis 40% mit verbesserten Geschmacks- und Tablettiereigenschaften für den Lebensmittelbereich.

6. Verwendung von Eiweißen bzw. Eiweißhydrolysaten nach Anspruch 5 mit einem Gehalt von 3 bis 15% oder Mineralsalzen mit einem Gehalt von 5 bis 20%.

## Claims

1. Xylitol- and sugar-free, vitamin-containing chewable tablet based on sorbitol for the foodstuffs sector, characterized in that it contains a softening agent in the form of proteins or protein hydrolysates having a content of 2 to 40 % or mineral salts from the group consisting of the carbonates, bicarbonates, phosphates, hydrogen phosphates, sulphates, chlorides and fluorides of sodium, potassium, calcium, magnesium or iron having a content of 5 to 40 %.

2. Chewable tablet according to Claim 1, having a content of proteins or protein hydrolysates of 3 to 15 % or a content of salts of 5 to 20 %.

3. Chewable tablet according to one of Claims 1 or 2, characterized in that it contains vitamins having a content of 0.1 to 10 %.

4. Process for the preparation of a chewable tablet according to one of Claims 1 to 3, characterized in that sorbitol is mixed with the softening agent, the vitamins and if appropriate with other substances permitted in accordance with the foodstuffs regulations and directly compressed to give the chewable tablet.

5. Use of proteins, protein hydrolysates or mineral salts as softening agents in xylitol- and sugar-free chewable tablets having a content of proteins of 2 to 40 % or a content of mineral salts according to Claim 1 of 5 to 40 % having improved flavour and tabletting properties for the foodstuffs sector.

6. Use of proteins or protein hydrolysates according to Claim 5 having a content of 3 to 15 % or mineral salts.

## Revendications

1. Tablette à mâcher, à base de sorbitol, sans xylitol ni sucres, contenant des vitamines, destinée au domaine alimentaire, caractérisée en ce qu'elle contient un désintégrant sous forme de protéines ou d'hydrolysats de protéines en une quantité de 2 à 40 %, ou des sels minéraux choisis parmi l'ensemble comprenant les carbonates, les bicarbonates, les phosphates, les hydrogénophosphates, les sulfates, les chlorures et les fluorures de sodium, de potassium, de calcium, de magnésium ou de fer en une quantité de 5 à 40 %.

2. Tablette à mâcher selon la revendication 1, contenant des protéines ou des hydrolysats de protéines en une quantité de 3 à 15 %, ou des sels en une quantité de 5 à 20 %.

3. Tablette à mâcher selon l'une des revendications 1 ou 2, caractérisée en ce qu'elle contient des vitamines en une quantité de 0,1 à 10 %.

4. Procédé de fabrication d'une tablette à mâcher selon l'une des revendications 1 à 3, caractérisé en ce qu'on mélange à du sorbitol le désintégrant, les vitamines et éventuellement d'autres substances autorisées par la législation sur les produits alimentaires, et qu'on les comprime directement pour donner une tablette à mâcher.

5. Utilisation de protéines, d'hydrolysats de protéines ou de sels minéraux, comme désintégrants dans des tablettes à mâcher sans xylitol ni sucres, contenant de 2 à 40 % de substances protéiques, ou contenant de 5 à 40 % de sels minéraux selon la revendication 1, présentant de meilleures propriétés de sapidité et de comprimage pour le domaine alimentaire.

6. Utilisation de protéines ou d'hydrolysats de protéines selon la revendication 5, en une quantité de 3 à 15 %, ou de sels minéraux en une quantité de 5 à 20 %.
